# EUROPEAN PATENT SPECIFICATION

(11) **EP 4 076 343 B1**
(45) Date of publication and mention of the grant of the patent: **08.11.2023**
(21) Application number: 20734566.1
(22) Date of filing: 30.06.2020
(51) Int. Cl.: A61K 8/04, A61K 8/06, A61K 8/33, A61K 8/34, A61K 8/37, A61K 8/39, A61K 8/44, A61K 8/67, A61Q 1/00, A61Q 19/08, A61K 8/81

(54) **RETINOL-BASED SERUM**
RETINOLBASIERTES SERUM
SÉRUM À BASE DE RÉTINOL

(30) Priority: 20.12.2019 FR 1915331
(43) Date of publication of application: 26.10.2022
(73) Proprietor: L'OREAL, 75008 Paris (FR)
(72) Inventor: RIMBERT, Sandrine, 94152 CHEVILLY LARUE (FR); FASTINGER, Xavier, 94152 CHEVILLY LARUE (FR); EYRAUD, Sonia, 94152 CHEVILLY LARUE (FR); BOISSARD, Charlotte, 94152 CHEVILLY LARUE (FR)
(74) Representative: Cabinet Nony
(86) International application number: PCT/EP2020/068389
(87) International publication number: WO 2021/121677

(56) References cited:
- EP-A1- 1 889 596
- WO-A2-96/07396
- FR-A1- 3 007 652
- TSUNODA T ET AL: "Stability of all-trans-retinol in cream", JOURNAL OF THE SOCIETY COSMETIC CHEMISTS, SOCIETY OF COSMETIC CHEMISTS, US, vol. 46, 1 July 1995 (1995-07-01), pages 191-198, XP002095668, ISSN: 0037-9832

## Description

### Technical field

The present invention relates to the field of caring for and/or making up keratin materials, in particular to the antiaging care of keratin materials, notably of the skin.

For the purposes of the present invention, the term *"keratin materials"* notably denotes the skin, the lips and/or the eyelashes, in particular the skin and/or the lips, and preferably the skin of the body and/or the face, and more preferentially of the face.

Skin aging results from the effects of intrinsic and extrinsic factors on the skin. During the aging process, a detrimental change in the structure and functions of the skin appears. The main clinical signs due to these modifications of the skin metabolism are the appearance of wrinkles and fine lines, the cause of which is a slackening and loss of the elasticity of the tissues.

Moreover, intrinsic aging, which brings about the changes in the skin, notably causes slowing-down of the renewal of the skin cells, which is reflected essentially by the appearance of detrimental clinical changes, such as a reduction in the subcutaneous adipose tissue and the appearance of small wrinkles or fine lines, and by histopathological changes, such as an increase in the number and thickness of elastic fibers, a loss of vertical fibers from the membrane of the elastic tissue, and the presence of large irregular fibroblasts in the cells of this elastic tissue.

### Prior art

It is known practice to treat these signs of skin aging using cosmetic or dermatological compositions containing active agents that are capable of combating aging, of which retinoids represent a known family of active agents.

Among the compounds of the retinoid family, retinol, which is one of the forms of vitamin A, is particularly interesting. Specifically, retinol is a natural endogenous constituent of the human body. Furthermore, it is well tolerated when applied to the skin up to much higher levels than for retinoic acid.

However, when it is introduced into a cosmetic or dermatological composition for topical application, the degradation of retinol is rapid, due to the effect of light, oxygen, metal ions, oxidizing agents, water or, in particular, due to an increase in temperature.

One of the signs of retinol degradation is the change in the odor of the cosmetic compositions in which it is used.

Specifically, retinol degradation also gives rise to the release of an odor which users find particularly unpleasant and inconvenient, and this can limit its use in cosmetic compositions. Thermal degradation of retinol was notably the subject of a study published in J. Soc. Cosm. Chem. 46, 191-198 (July-August 1995).

As a result, retinol formulated in cosmetic compositions is unstable, and it has already been proposed to stabilize the retinol contained in these formulations.

For example, WO 96/07396 suggests the stabilization of retinol in a cosmetic composition in the form of an oil-in-water emulsion, by using at least one liposoluble antioxidant active agent and/or at least one sequestrant. An example of an antioxidant active agent that is mentioned is butyl hydroxy toluene (BHT). Moreover, ethylenediaminetetraacetic acid (EDTA) and derivatives thereof are mentioned as sequestrants.

However, BHT is a controversial compound, as it is potentially toxic to human beings. In addition, EDTA and BHT are neither environmentally friendly nor biodegradable. Furthermore, this system does not solve the problem of the change in the odor of the composition.

Moreover, WO 93/00085 describes water-in-oil emulsions comprising retinol and a stabilizing system consisting of a chelating agent, for instance EDTA, and an antioxidant. According to said document, water-in-oil emulsions containing retinol stabilized with a system consisting of a liposoluble antioxidant and of a water-soluble antioxidant may also be prepared.

However, although said document teaches that the combination of EDTA and BHT can stabilize retinol in a composition in the form of water-in-oil emulsions, such a combination appears to be ineffective when the composition is formulated in the form of an oil-in-water emulsion.

Thus, despite the use of antioxidants and chelating agents, the retinol degrades over time, which results in an unpleasant odor for the users of cosmetic compositions.

### Presentation of the invention

There is thus still a need for cosmetic compositions containing retinol, which are notably efficient for combating the signs of aging of keratin materials and which make it possible to control retinol degradation and the change in the composition's odor over time.

There is also a need for such cosmetic compositions which are compatible with consumers' requirements, notably regarding the environment.

Moreover, there is still a need for efficient stabilization of retinol in a cosmetic composition, and for control of the change in the odor of this composition over time, regardless of the galenic form of the composition.

The invention is specifically directed toward meeting these needs.

### Summary of the invention

Therefore, according to a first of its aspects, the present invention relates to a composition, notably a cosmetic composition, in particular for making up and/or caring for keratin materials, in the form of an oil-in-water emulsified gel, comprising:
- retinol;
- di-t-butyl pentaerythrityl tetrahydroxycinnamate;
- at least one ethylenediaminedisuccinic acid salt;
- at least 11% by weight of at least one polyol relative to the total weight of the composition;
- at least one hydrophilic gelling agent;
- at least one derivative of polyethylene glycol and of mono-, di- and tri-glycerides of an acid including at least one C₆ to C₁₆ alkyl chain, and containing at least two ethylene oxide groups;
- at least one non-volatile ether oil containing from 10 to 40 carbon atoms; and
- at least one non-volatile monoester oil.

The inventors have observed, surprisingly, that the combination of at least one di-t-butyl pentaerythrityl tetrahydroxycinnamate and of an ethylenediaminedisuccinic acid salt in a specific galenic form makes it possible to efficiently stabilize the retinol contained in a cosmetic composition and to control the change in its odor over time.

Specifically, as shown in the examples hereinbelow, the compositions according to the invention are stable, and the control of the change in their odor over time is able to be significantly improved.

It has thus been observed that the compositions in accordance with the invention are stable while at the same time having organoleptic properties which are pleasant for the user, in particular an odor and a color which meet the users' expectations. In addition, no degradation of the odor or color occurs when using the composition.

Advantageously, a composition according to the invention comprises a low content of controversial ingredients, and in particular comprises less than 0.2% by weight of butyl hydroxy toluene (BHT) and/or ethylenediaminetetraacetic acid (EDTA) or a derivative thereof, and preferably does not comprise any controversial ingredients, and in particular does not comprise either butyl hydroxytoluene (BHT) or ethylenediaminetetraacetic acid (EDTA) or a derivative thereof.

According to another one of its aspects, the invention also relates to a cosmetic, non-therapeutic process for making up and/or caring for, preferably caring for, keratin materials, in particular the skin and/or the lips, comprising at least one step of applying a composition according to the invention to said keratin materials.

Said cosmetic process for making up and/or caring for, preferably caring for, keratin materials, in particular the skin and/or the lips, is non-therapeutic.

Other features, variants and advantages of the compositions according to the invention will emerge more clearly on reading the description and the examples that follow.

### Detailed description

### Retinol

As stated previously, a composition according to the invention comprises retinol, also known as vitamin A.

For the purposes of the present invention, the term *"retinol"* denotes all the isomers of retinol, notably all-trans retinol, 13-cis retinol, 11-cis retinol, 9-cis retinol and 3.4-didehydroretinol.

Preferably, all-trans retinol is used.

In particular, a composition according to the invention comprises an effective amount of retinol.

For the purposes of the present invention, the term *"effective amount"* denotes an amount of retinol which results in the desired effect via its implementation, notably a reduction in the signs of aging of the keratin materials.

Preferably, a composition according to the invention comprises at least 0.02% by weight of retinol relative to the total weight of the composition.

More particularly, a composition according to the invention may comprise an amount of retinol of between 0.02% and 5.0% by weight, notably between 0.05% and 3.0% by weight, preferably between 0.08% and 1.0% by weight, and more preferentially between 0.1% and 0.5% by weight, relative to the total weight of the composition.

It is understood that the retinol content corresponds to the content of active material, also known as the solids content, of retinol introduced into the composition.

According to a particular embodiment variant, retinol may be introduced into the composition in a form which is dissolved in an oil, such as a plant oil, for example soybean oil, notably in a content ranging from 5% to 20% by weight, preferably of about 10% by weight in the oil.

Those sold by the company BASF, notably under the name Retinol 10SU, in a content of 10% by weight of active material in soybean oil, are most particularly suitable for use. According to another particular embodiment variant, an encapsulated form of retinol may also be used.

### Di-t-butyl pentaerythrityl tetrahydroxycinnamate

A composition according to the invention also comprises at least di-t-butyl pentaerythrityl tetrahydroxycinnamate.

Di-t-butyl pentaerythrityl tetrahydroxycinnamate or tetra-di-t-butyl pentaerythrityl hydroxyhydrocinnamate is a compound which belongs to the family of cinnamic acids and derivatives thereof, the CAS number of which is 6683-19-8.

By way of example, mention may be made of the di-t-butyl pentaerythrityl tetrahydroxycinnamate compound sold by the company BASF under the name Tinogard TT^{®}.

Preferably, a composition according to the invention comprises from 0.01% to 2.5% by weight, preferably from 0.05% to 1.5% by weight and more preferentially from 0.1% to 0.3% by weight of di-t-butyl pentaerythrityl tetrahydroxycinnamate, relative to the total weight of the composition.

### Ethylenediaminedisuccinic acid salt

A composition according to the invention also comprises at least one ethylenediaminedisuccinic acid salt.

Ethylenediaminedisuccinic acid is a compound of formula:

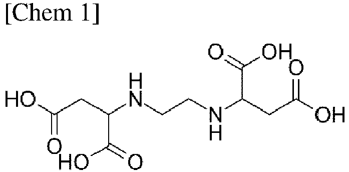

Preferably, the ethylenediaminedisuccinic acid salt is chosen from alkali metal salts, such as potassium salts and sodium salts, ammonium salts, and amine salts. Alkali metal salts of ethylenediaminedisuccinic acid are more particularly preferred.

Preferably, the ethylenediaminedisuccinic acid salt used according to the invention is trisodium ethylenediaminedisuccinate.

Such a compound is, for example, the compound sold under the name Natrlquest^{®} E30 by the company Innospec Active Chemicals, or the compound sold under the name Octaquest E30^{®} by the company Octel Performance Chemicals.

Preferably, a composition according to the invention comprises from 0.01% to 2.5% by weight, preferably from 0.05% to 1.5% by weight and more preferentially from 0.07% to 0.3% by weight of ethylenediaminedisuccinic acid salt, relative to the total weight of the composition.

It is understood that the content of ethylenediaminedisuccinic acid salt corresponds to the content of active material, also known as the solids content, of ethylenediaminedisuccinic acid salt introduced into the composition.

According to a particular embodiment variant, the ethylenediaminedisuccinic acid salt may be introduced into the composition in a form which is dissolved in water, notably in a content ranging from 25% to 50% by weight, preferably from 35% to 40% by weight in water.

Such a compound is, for example, the compound sold under the name Natrlquest^{®} E30 by the company Innospec Active Chemicals, at 37% by weight in water.

According to one embodiment, a composition according to the invention comprises at least one di-t-butyl pentaerythrityl tetrahydroxycinnamate compound and at least one ethylenediaminedisuccinic acid salt in a mass ratio of di-t-butyl pentaerythrityl tetrahydroxycinnamate compound / ethylenediaminedisuccinic acid salt of from 1.5 to 5, and preferably from 2.5 to 4.5.

### Polyol

A composition according to the invention comprises at least 11% by weight of at least one polyol relative to the total weight of the composition.

The polyol(s) may be present in a composition according to the invention in a content ranging from 11% to 25% by weight, preferably from 13% to 20% by weight and more preferentially from 14% to 18% relative to the total weight of the composition.

For the purposes of the present invention, the term *"polyol"* should be understood as meaning any organic molecule including at least two free hydroxyl groups.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are suitable for formulating a composition according to the present invention are in particular those notably containing from 2 to 32 carbon atoms and preferably 2 to 16 carbon atoms.

The polyol may be chosen, for example, from ethylene glycol, pentaerythritol, trimethylolpropane, propylene glycol, 1,3-propanediol, butylene glycol, isoprene glycol, pentylene glycol, hexylene glycol, glycerol, polyglycerols, such as glycerol oligomers, for instance diglycerol, and polyethylene glycols, and mixtures thereof.

According to a preferred embodiment of the invention, the polyol may be chosen, for example, from ethylene glycol, propylene glycol, 1,3-propanediol, 1,3-butylene glycol, isoprene glycol, 1,2-pentylene glycol, hexylene glycol, caprylyl glycol, dipropylene glycol, glycerol, pentaerythritol, trimethylolpropane, polyglycerols, polyethylene glycols, and mixtures thereof.

According to a preferred embodiment, the composition of the invention comprises at least glycerol.

According to a preferred embodiment, a composition according to the invention may comprise at least two different polyols, and preferably at least three different polyols.

Preferably, it comprises at least glycerol and at least one different polyol chosen from ethylene glycol, propylene glycol, 1,3-propanediol, 1,3-butylene glycol, isoprene glycol, 1,2-pentylene glycol, hexylene glycol, caprylyl glycol, dipropylene glycol, and mixtures thereof.

Preferably, a composition according to the invention may comprise from 3% to 8% by weight of glycerol and from 8% to 15% by weight of at least one different polyol chosen from ethylene glycol, propylene glycol, 1,3-propanediol, 1,3-butylene glycol, isoprene glycol, 1,2-pentylene glycol, hexylene glycol, caprylyl glycol, dipropylene glycol, and mixtures thereof, relative to the total weight of the composition.

### Hydrophilic gelling agent

A composition according to the invention is in the form of an oil-in-water emulsified gel and comprises at least one hydrophilic gelling agent.

For the purposes of the present invention, the term *"hydrophilic gelling agent"* means a compound that is capable of gelling the aqueous phase of the compositions according to the invention.

The gelling agent is hydrophilic and is thus present in the aqueous phase of the composition. The gelling agent may be water-soluble or water-dispersible.

As stated above, the aqueous phase of a composition according to the invention is gelled with at least one hydrophilic gelling agent.

The hydrophilic gelling agent may be chosen from synthetic polymeric gelling agents, polymeric gelling agents that are natural or of natural origin, mixed silicates and fumed silicas, and mixtures thereof.

Preferably, the hydrophilic gelling agent may be chosen from synthetic polymeric gelling agents, polymeric gelling agents that are natural or of natural origin, and mixtures thereof. More preferentially, the hydrophilic gelling agent may be chosen from synthetic polymeric gelling agents.

According to a preferred embodiment, a composition according to the invention comprises at least two different hydrophilic gelling agents.

Preferably, the hydrophilic gelling agent(s) are chosen from crosslinked and/or neutralized polymers and copolymers of 2-acrylamido-2-methylpropanesulfonic acid, polysaccharides, and mixtures thereof.

### I. Polymeric gelling agents that are natural or of natural origin

The polymeric hydrophilic gelling agents that are suitable for use in the invention may be natural or of natural origin.

For the purposes of the invention, the term *"of natural origin"* is intended to denote polymeric gelling agents obtained by modification of natural polymeric gelling agents.

These gelling agents may be particulate or non-particulate.

More specifically, these gelling agents fall within the category of polysaccharides.

A composition according to the invention may comprise a gelling agent of polysaccharide type in a content ranging from 0.05% to 5.0% by weight, notably from 0.1% to 2.5% by weight, preferably between 0.1% and 2.0% by weight, in particular of about 0.4% by weight, relative to the total weight of the composition.

More specifically, these gelling agents fall within the category of polysaccharides.

In general, polysaccharides may be divided into several categories.

Thus, polysaccharides that are suitable for use in the invention may be homopolysaccharides such as fructans, glucans, galactans and mannans or heteropolysaccharides such as hemicellulose.

Similarly, they may be linear polysaccharides such as pullulan or branched polysaccharides such as acacia gum and amylopectin, or mixed polysaccharides such as starch.

More particularly, the polysaccharides that are suitable for use in the invention may be distinguished according to whether or not they are starchy.

As representatives of the starchy polysaccharides, mention may be made most particularly of native starches, modified starches and particulate starches.

According to one embodiment variant, the hydrophilic gelling agent is non-starchy.

In general, the non-starchy polysaccharides may be chosen from polysaccharides produced by microorganisms; polysaccharides isolated from algae, and higher plant polysaccharides, such as homogeneous polysaccharides, in particular celluloses and derivatives thereof or fructosans, heterogeneous polysaccharides such as acacia gums, galactomannans, glucomannans and pectins, and derivatives thereof; and mixtures thereof.

In particular, the polysaccharides may be chosen from fructans, gellans, glucans, amylose, amylopectin, glycogen, pullulan, dextrans, celluloses and derivatives thereof, in particular methylcelluloses, hydroxyalkylcelluloses, ethylhydroxyethylcelluloses and carboxymethylcelluloses, mannans, xylans, lignins, arabans, galactans, galacturonans, alginate-based compounds, chitin, chitosans, glucuronoxylans, arabinoxylans, xyloglucans, glucomannans, pectic acids and pectins, arabinogalactans, carrageenans, agars, glycosaminoglucans, acacia gums, tragacanth gums, ghatti gums, karaya gums, locust bean gums, galactomannans such as guar gums and nonionic derivatives thereof, in particular hydroxypropyl guar, and ionic derivatives thereof, biopolysaccharide gums of microbial origin, in particular scleroglucan or xanthan gums, mucopolysaccharides, and in particular chondroitin sulfates, and mixtures thereof.

These polysaccharides may be chemically modified, notably with urea or urethane groups or by hydrolysis, oxidation, esterification, etherification, sulfatation, phosphatation, amination, amidation or alkylation reaction, or by several of these modifications.

The derivatives obtained may be anionic, cationic, amphoteric or nonionic.

Advantageously, the polysaccharides may be chosen from carrageenans, in particular kappa carrageenan, gellan gum, agar-agar, xanthan gum, alginate-based compounds, in particular sodium alginate, scleroglucan gum, guar gum, inulin and pullulan, and mixtures thereof.

Preferably, the polysaccharide may be xanthan gum.

Xanthan is a heteropolysaccharide produced on an industrial scale by the aerobic fermentation of the bacterium *Xanthomonas campestris.* Its structure consists of a main chain of β(1,4)-linked (β-D-glucoses, similar to cellulose. One glucose molecule in two bears a trisaccharide side chain composed of an α-D-mannose, a β-D-glucuronic acid and a terminal β-D-mannose. The internal mannose residue is generally acetylated on carbon 6. About 30% of the terminal mannose residues bear a pyruvate group linked in chelated form between carbons 4 and 6. The charged pyruvic acids and glucuronic acids are ionizable, and are thus responsible for the anionic nature of xanthan (negative charge down to a pH equal to 1). The content of the pyruvate and acetate residues varies according to the bacterial strain, the fermentation process, the conditions after fermentation and the purification steps. These groups may be neutralized in commercial products with Na⁺, K⁺ or Ca²⁺ ions (Satia company, 1986). The neutralized form may be converted into the acid form by ion exchange or by dialysis of an acidic solution.

Xanthan gums have a molecular weight of between 1000 000 and 50 000 000 and a viscosity of between 0.6 and 1.65 Pa.s for an aqueous composition containing 1% of xanthan gum (measured at 25°C on a Brookfield viscometer of LVT type at 60 rpm).

Xanthan gums are represented, for example, by the products sold under the names Rhodicare by the company Rhodia Chimie, under the name Satiaxane^{™} by the company Cargill Texturizing Solutions (for the food, cosmetic and pharmaceutical industries), under the name Novaxan^{™} by the company ADM, and under the names Kelzan^{®} and Keltrol^{®} by the company CP-Kelco.

Advantageously, a composition according to the invention comprises a xanthan gum.

Such a gelling agent may be used in a proportion of from 0.1% to 8.0% by weight of solids relative to the total weight of the aqueous phase, notably from 0.1% to 6.0% by weight, preferably between 0.5% and 2.5% by weight and in particular in a proportion of about 1.5%, or alternatively in a proportion of about 1% by weight, relative to the total weight of the aqueous phase.

Among the other polysaccharides that may be used according to the invention, mention may also be made of chitin (poly-N-acetyl-D-glucosamine, β(1,4)-2-acetamido-2-deoxy-D-glucose), chitosan and derivatives (chitosan-beta-glycerophosphate, carboxymethylchitin, etc.) such as those sold by the company France-Chitine; glycosaminoglycans (GAG) such as hyaluronic acid, chondroitin sulfate, dermatan sulfate, keratan sulfate, and preferably hyaluronic acid; xylans (or arabinoxylans) and derivatives.

Arabinoxylans are polymers of xylose and arabinose, all grouped under the name *pentosans.* Xylans are constituted of a main chain of β(1,4) linked D-xylose units and on which are found three substituents (Rouau & Thibault, 1987): acid units, α-L-arabinofuranose units, side chains which may contain arabinose, xylose, galactose and glucuronic acid.

According to this variant, the polysaccharide is preferably hyaluronic acid, or a salt thereof such as the sodium salt (sodium hyaluronate).

### II. Synthetic polymeric gelling agents

For the purposes of the invention, the term "synthetic" means that the polymer is neither naturally existing nor a derivative of a polymer of natural origin.

The synthetic polymeric hydrophilic gelling agent under consideration according to the invention may or may not be particulate.

For the purposes of the invention, the term "particulate" means that the polymer is in the form of particles, preferably spherical particles.

As emerges from the text hereinbelow, the polymeric hydrophilic gelling agent is advantageously chosen from crosslinked acrylic homopolymers or copolymers; associative polymers, in particular associative polymers of polyurethane type; polyacrylamides and crosslinked and/or neutralized 2-acrylamido-2-methylpropanesulfonic acid polymers and copolymers; modified or unmodified carboxyvinyl polymers, and mixtures thereof, notably as defined below.

According to a preferred embodiment, the hydrophilic gelling agent is chosen from synthetic polymeric gelling agents, and preferably from associative polymers, polyacrylamides, 2-acrylamido-2-methylpropanesulfonic acid polymers and copolymers and carboxyvinyl polymers, and more preferentially from 2-acrylamido-2-methylpropanesulfonic acid polymers and copolymers.

More preferentially, the hydrophilic gelling agent is chosen from crosslinked or noncrosslinked sodium acrylamido-2-methylpropanesulfonate polymers.

The particulate synthetic polymeric gelling agents are preferably chosen from crosslinked polymers.

They may notably be crosslinked acrylic homopolymers or copolymers, which are preferably partially neutralized or neutralized, and which are in particulate form.

According to one embodiment, the particulate gelling agent according to the present invention is chosen from crosslinked sodium polyacrylates.

The family of non-particulate synthetic polymeric gelling agents may be detailed as the following subfamilies: associative polymers, polyacrylamides and crosslinked and/or neutralized 2-acrylamido-2-methylpropanesulfonic acid polymers and copolymers, and modified or unmodified carboxyvinyl polymers.

For the purposes of the present invention, the term *"associative polymer"* means any amphiphilic polymer including in its structure at least one fatty chain and at least one hydrophilic portion. The associative polymers in accordance with the present invention may be anionic, cationic, nonionic or amphoteric.

The polymers used that are suitable as aqueous gelling agent for the invention may be crosslinked or non-crosslinked homopolymers or copolymers including at least the 2-acrylamido-2-methylpropanesulfonic acid (AMPS^{®}) monomer, in a form partially or totally neutralized with a mineral base other than aqueous ammonia, such as sodium hydroxide or potassium hydroxide. They are preferably totally or almost totally neutralized, i.e. at least 90% neutralized.

These AMPS^{®} polymers according to the invention may be crosslinked or non-crosslinked. The AMPS^{®} polymers that are suitable for use in the invention are water-soluble or water-dispersible.

The term *"water-soluble or water-dispersible"* means polymers which, when introduced into an aqueous phase at 25°C, at a mass concentration equal to 1%, make it possible to obtain a macroscopically homogeneous and transparent solution, i.e. a solution with a maximum light transmittance value, at a wavelength equal to 500 nm, through a sample 1 cm thick, of at least 60% and preferably of at least 70%.

In this case, they are either "homopolymers" including only AMPS monomers and, if they are crosslinked, one or more crosslinking agents such as those defined above, or copolymers obtained from AMPS^{®} and one or more hydrophilic or hydrophobic ethylenically unsaturated monomers and, if they are crosslinked, one or more crosslinking agents. When said copolymers include hydrophobic ethylenically unsaturated monomers, these monomers do not include a fatty chain and are preferably present in small amounts.

The water-soluble or water-dispersible AMPS^{®} polymers of the invention preferably have a molar mass ranging from 50 000 g/mol to 10 000 000 g/mol, preferably from 80 000 g/mol to 8 000 000 g/mol, and even more preferably from 100 000 g/mol to 7 000 000 g/mol.

As water-soluble or water-dispersible AMPS homopolymers that are suitable for use in the invention, mention may be made, for example, of crosslinked or non-crosslinked polymers of sodium acrylamido-2-methylpropanesulfonate, such as that used in the commercial product Simulgel 800 (CTFA name: Sodium Polyacryloyldimethyl Taurate), crosslinked ammonium acrylamido-2-methylpropanesulfonate polymers (INCI name: Ammonium Polyacryldimethyltauramide) such as those described in patent EP 0 815 928 B1 and such as the product sold under the trade name Hostacerin AMPS^{®} by the company Clariant.

As water-soluble or water-dispersible AMPS copolymers in accordance with the invention, examples that may be mentioned include:
- crosslinked acrylamide/sodium acrylamido-2-methylpropanesulfonate copolymers, such as that used in the commercial product Sepigel 305 (CTFA name: Polyacrylamide/C₁₃-C₁₄ isoparaffin/laureth-7) or that used in the commercial product sold under the name Simulgel 600 (CTFA name: Acrylamide/sodium acryloyldimethyltaurate copolymer/isohexadecane/polysorbate-80) by the company SEPPIC;
- copolymers of AMPS^{®} and of vinylpyrrolidone or vinylformamide, such as that used in the commercial product sold under the name Aristoflex AVC^{®} by the company Clariant (CTFA name: Ammonium Acryloyldimethyltaurate/VP copolymer) but neutralized with sodium hydroxide or potassium hydroxide;
- copolymers of AMPS^{®} and of sodium acrylate, for instance the AMPS/sodium acrylate copolymer, such as that used in the commercial product sold under the name Simulgel EG^{®} by the company SEPPIC or under the trade name Sepinov EM (CTFA name: Hydroxyethyl acrylate/sodium acryloyldimethyltaurate copolymer);
- copolymers of AMPS^{®} and of hydroxyethyl acrylate, for instance the AMPS^{®}/hydroxyethyl acrylate copolymer, such as that used in the commercial product sold under the name Simulgel NS^{®} by the company SEPPIC (CTFA name: Hydroxyethyl acrylate/sodium acryloyldimethyltaurate copolymer (and) squalane (and) polysorbate 60), or such as the product sold under the name sodium acrylamido-2-methylpropanesulfonate/hydroxyethyl acrylate copolymer, such as the commercial product Sepinov EMT 10 (INCI name: Hydroxyethyl acrylate/sodium acryloyldimethyltaurate copolymer).

As preferred water-soluble or water-dispersible AMPS copolymers in accordance with the invention, mention may be made of copolymers of AMPS^{®} and of hydroxyethyl acrylate.

Preferably, a composition according to the invention comprises an AMPS^{®} homopolymer, preferably a crosslinked polymer of ammonium acrylamido-2-methylpropanesulfonate.

In particular, such an AMPS^{®} homopolymer may be present in a composition according to the invention in a content of between 0.01% and 5.0% by weight, preferably between 0.1% and 3.0% by weight, and more preferentially between 0.2% and 2.5% by weight, relative to the total weight of the composition.

Advantageously, a composition according to the invention comprises a synthetic polymeric hydrophilic gelling agent chosen from 2-acrylamido-2-methylpropanesulfonic acid polymers and copolymers.

According to a preferred variant, the synthetic polymeric hydrophilic gelling agent is an AMPS^{®} homopolymer.

### III. Other hydrophilic gelling agents

As other hydrophilic gelling agents, mention may be made more particularly of mixed silicates and fumed silicas.

The mixed silicates that are suitable for use in the invention may be chosen, for example, from montmorillonites, hectorites, bentonites, beidellite and saponites. According to a preferred embodiment of the invention, the mixed silicates used are more particularly chosen from hectorites and bentonites, and better still from laponites.

The hydrophilic fumed silicas are obtained by pyrolysis of silicon tetrachloride (SiCl₄) in a continuous flame at 1000°C in the presence of hydrogen and oxygen. Among the fumed silicas of hydrophilic nature that may be used according to the present invention, mention may notably be made of those sold by the company Degussa or Evonik Degussa under the trade names Aerosil^{®} 90, 130, 150, 200, 300 and 380 or by the company Cabot under the name Carbosil H5.

According to a preferred embodiment, a composition according to the invention comprises at least one hydrophilic gelling agent chosen from polysaccharides, in particular polysaccharides synthesized by microorganisms, non-particulate synthetic polymeric gelling agents, in particular crosslinked and/or neutralized 2-acrylamido-2-methylpropanesulfonic acid polymers and copolymers, and mixtures thereof.

The polysaccharides synthesized by microorganisms that are preferred according to the invention are chosen from xanthan gum, sclerotium gum, pullulan, and mixtures thereof. It may be the Xanthan Gum (and) Sclerotium (and) Lecithin (and Pullulan) mixture sold under the name Siligel^{®}.

According to a preferred embodiment, a composition according to the invention comprises at least two different hydrophilic gelling agents, or even at least three different hydrophilic gelling agents.

A composition according to the invention may comprise between 0.05% and 5.0% by weight of hydrophilic gelling agent(s), preferably between 0.1% and 2.5% by weight, more preferentially between 0.5% and 1.5% by weight, relative to the total weight of said composition.

### Derivative of polyethylene glycol and of glyceride of an acid

A composition according to the invention comprises at least one derivative of polyethylene glycol and of mono-, di- and tri-glycerides of an acid including at least one C₆ to C₁₆ alkyl chain, and containing at least two ethylene oxide groups.

The derivatives of polyethylene glycol and of mono-, di- and tri-glycerides of an acid preferably include at least one C₆ to C₁₄ alkyl chain, and contain at least two ethylene oxide groups, and preferably from 6 to 10 ethylene oxide groups.

As examples of such derivatives, mention may be made of mono-, di- and tri-glycerides of caprylic acid and of capric acid, such as those including 6 ethylene oxide groups (INCI name: PEG-6 caprylic/capric glycerides), sold under the name Softigen 767 by the company Sasol. The derivative(s) of polyethylene glycol and of mono-, di- and tri-glycerides of an acid including at least one C₆ to C₁₆ alkyl chain, and containing at least two ethylene oxide groups may be present in a composition according to the invention in a content ranging from 0.2% to 10% by weight, preferably from 0.5% to 5.0% by weight, more preferentially from 0.5% to 2.5% by weight, relative to the total weight of the composition.

### Ether oil

A composition according to the invention also comprises at least one non-volatile ether oil containing from 10 to 40 carbon atoms.

For the purposes of the present invention, the term *"non-volatile oil"* denotes an oil having a vapor pressure at room temperature and atmospheric pressure which is nonzero and less than 10⁻³ mmHg (0.13 Pa).

Such oils are hydrocarbon-based oils, i.e. they contain mainly hydrogen and carbon atoms. Preferably, the non-volatile ether oil that is suitable for use in the invention comprises from 10 to 30 carbon atoms, preferably from 12 to 22 carbon atoms.

As an example of ethers containing from 10 to 40 carbon atoms, mention may be made of dicaprylyl ether (CTFA name: Dicaprylyl ether).

Such a non-volatile ether oil may be present in a composition according to the invention in a content ranging from 0.5% to 10% by weight, preferably from 1.0% to 5.0% by weight and more particularly from 2.0% to 4.0% by weight relative to the total weight of the composition.

### Monoester oil

A composition according to the invention also comprises at least one non-volatile monoester oil.

Such a monoester oil may notably comprise between 18 and 70 carbon atoms, preferably between 18 and 40 carbon atoms.

In particular, the volatile monoester oil in accordance with the invention is of formula R₁COOR₂ in which R₁ represents a linear or branched fatty acid residue including from 4 to 40 carbon atoms and R₂ represents a hydrocarbon-based chain, which is notably branched, containing from 4 to 40 carbon atoms, on condition that R₁ + R₂ is greater than or equal to 18, for instance Purcellin oil (cetostearyl octanoate), isononyl isononanoate, C₁₂ to C₁₅ alkyl benzoates, 2-ethylhexyl palmitate, octyldodecyl neopentanoate, 2-octyldodecyl stearate, 2-octyldodecyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate or 2-diethylhexyl succinate.

Preferably, they are esters of formula R₁COOR₂ in which R₁ represents a linear or branched fatty acid residue including from 4 to 40 carbon atoms and R₂ represents a hydrocarbon-based chain that is notably branched, containing from 4 to 40 carbon atoms, R₁ and R₂ being such that R₁ + R₂ is greater than or equal to 18.

Preferred monoesters that may be mentioned include isononyl isononanoate, oleyl erucate and/or 2-octyldodecyl neopentanoate, preferably isononyl isononanoate.

The volatile monoester oil may be present in a composition according to the invention in a content ranging from 0.5% to 10% by weight, preferably from 1.0% to 5.0% by weight and more preferentially from 2.0% to 4.0% by weight relative to the total weight of the composition.

According to a preferred embodiment, a composition according to the invention, in particular a cosmetic composition according to the invention, comprises at least:
- between 0.05% and 1% by weight of retinol;
- between 0.05% and 1.5% by weight of di-t-butyl pentaerythrityl tetrahydroxycinnamate,
- between 0.05% and 1.5% by weight of ethylenediaminedisuccinic acid salt,
- between 11% and 25% by weight of at least one polyol;
- between 0.1% and 1.0% by weight of at least one hydrophilic gelling agent;
- between 0.5% and 2.5% by weight of at least one derivative of polyethylene glycol and of mono-, di- and tri-glycerides of an acid including at least one C₆ to C₁₆ alkyl chain, and containing at least two ethylene oxide groups;
- between 1.0% and 5.0% by weight of at least one nonvolatile ether oil containing from 10 to 40 carbon atoms; and
- between 1.0% and 5.0% by weight of at least one nonvolatile monoester oil, relative to the total weight of the composition.

### Aqueous phase

The aqueous phase comprises water and optionally a water-soluble solvent, other than the polyols defined above.

It is understood that the hydrophilic gelling agent and the polyols used according to the invention are also present in the aqueous phase.

According to the present invention, the term *"water-soluble solvent"* denotes a compound that is liquid at room temperature and water-miscible (miscibility with water of greater than 50% by weight at 25°C and atmospheric pressure).

The water-soluble solvents that may be used in the composition of the invention may also be volatile.

Among the water-soluble solvents that may be used in the composition according to the invention, mention may be made notably of lower monoalcohols containing from 1 to 5 carbon atoms, such as ethanol and isopropanol, C₃ and C₄ ketones and C₂-C₄ aldehydes.

Preferably, the aqueous phase is present in a composition according to the invention in a content ranging from 50% to 95% by weight and preferably from 60% to 90% by weight relative to the total weight of said composition.

### Oily phase

A composition according to the invention is in the form of an oil-in-water emulsified gel, and thus includes an oily phase.

It is understood that the derivative(s) of polyethylene glycol and of mono-, di- and triglycerides of an acid including at least one C₆ to C₁₆ alkyl chain, and containing at least two ethylene oxide groups, the non-volatile ether oil(s) containing from 10 to 40 carbon atoms and the non-volatile monoesters oil(s) are present in the oily phase.

The oily phase may also comprise oils other than the previously mentioned oils, notably cosmetic oils. It may also contain other fatty substances.

The term *"oil"* means a water-immiscible nonaqueous compound that is liquid at room temperature (20°C) and at atmospheric pressure (760 mmHg).

An oily phase that is suitable for preparing the compositions, notably the cosmetic compositions, according to the invention may comprise hydrocarbon-based oils, silicone oils, fluoro oils or nonfluoro oils, or mixtures thereof.

The oils may be volatile or non-volatile.

They may be of animal, plant, mineral or synthetic origin.

For the purposes of the present invention, the term *"silicone oil"* means an oil comprising at least one silicon atom, and notably at least one Si-O group.

The term *"fluoro oil"* means an oil comprising at least one fluorine atom.

The term *"hydrocarbon-based oil"* means an oil mainly containing hydrogen and carbon atoms.

The oils may optionally comprise oxygen, nitrogen, sulfur and/or phosphorus atoms, for example in the form of hydroxyl or acid radicals.

For the purposes of the invention, the term *"volatile oil"* means any oil that is capable of evaporating on contact with the skin in less than one hour, at room temperature and atmospheric pressure. The volatile oil is a volatile cosmetic compound, which is liquid at room temperature, notably having a nonzero vapor pressure, at room temperature and atmospheric pressure, notably having a vapor pressure ranging from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

### Volatile oils

The volatile oils may be hydrocarbon-based oils or silicone oils.

Among the volatile hydrocarbon-based oils containing from 8 to 16 carbon atoms, mention may be made notably of branched C₈-C₁₆ alkanes, for instance C₈-C₁₆ isoalkanes (also known as isoparaffins), isododecane, isodecane, isohexadecane and, for example, the oils sold under the trade names Isopar or Permethyl, branched C₈-C₁₆ esters, for instance isohexyl neopentanoate, and mixtures thereof. In particular, the volatile hydrocarbon-based oil is chosen from volatile hydrocarbon-based oils containing from 8 to 16 carbon atoms and mixtures thereof.

Mention may also be made of volatile linear alkanes comprising from 8 to 16 carbon atoms, in particular from 10 to 15 carbon atoms and more particularly from 11 to 13 carbon atoms, for instance n-dodecane (C₁₂) and n-tetradecane (C₁₄) sold by Sasol under the respective references Parafol 12-97 and Parafol 14-97, and also mixtures thereof, the undecanetridecane mixture, mixtures of n-undecane (Cn) and of n-tridecane (C₁₃) obtained in Examples 1 and 2 of patent application WO 2008/155 059 from the company Cognis, and mixtures thereof.

Volatile silicone oils that may be mentioned include linear volatile silicone oils such as hexamethyldisiloxane, octamethyltrisiloxane, decamethyltetrasiloxane, tetradecamethylhexasiloxane, hexadecamethylheptasiloxane and dodecamethylpentasiloxane.

Volatile cyclic silicone oils that may be mentioned include hexamethylcyclotrisiloxane, octamethylcyclotetrasiloxane, decamethylcyclopentasiloxane, cyclohexasiloxane and dodecamethylcyclohexasiloxane, in particular cyclohexasiloxane.

### Non-volatile oils

The non-volatile oils may notably be chosen from non-volatile hydrocarbon-based, fluoro and/or silicone oils.

Non-volatile hydrocarbon-based oils that may notably be mentioned include:
- hydrocarbon-based oils of animal origin,
- hydrocarbon-based oils of plant origin,
- polyol esters and pentaerythritol esters, for instance dipentaerythrityl tetrahydroxystearate/tetraisostearate,
- fatty alcohols that are liquid at room temperature, with a branched and/or unsaturated carbon-based chain containing from 12 to 26 carbon atoms, for instance 2-octyldodecanol, isostearyl alcohol and oleyl alcohol,
- C₁₂-C₂₂ higher fatty acids, such as oleic acid, linoleic acid, linolenic acid, and mixtures thereof,
- carbonates, such as dicaprylyl carbonate,
- nonphenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, and trimethylpentaphenyltrisiloxane, and mixtures thereof;
and also mixtures of these various oils.

In particular, a composition according to the invention may comprise a non-volatile oil other than the non-volatile ether oil containing from 10 to 40 carbon atoms and the non-volatile monoester oil, and in particular chosen from non-volatile oils such as carbonates, and preferably is dicaprylyl carbonate.

For the purposes of the present invention, the term *"apolar oil"* means an oil whose solubility parameter at 25°C, δₐ, is equal to 0 (J/cm³)^{1/2}.

The definition and calculation of the solubility parameters in the Hansen three-dimensional solubility space are described in the article by C.M. Hansen: "The three dimensional solubility parameters" J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation δₐ = (δp² + 6h²)^{1/2}.

The parameters δₚ, δₕ, δ_{D} and δₐ are expressed in (J/cm³)^{1/2}.

In particular, the non-volatile apolar hydrocarbon-based oil is free of oxygen atoms.

Preferably, the non-volatile apolar hydrocarbon-based oil may be chosen from linear or branched hydrocarbons of mineral or synthetic origin. In particular, it may be chosen from:
- liquid paraffin or derivatives thereof,
- liquid petroleum jelly,
- naphthalene oil,
- polybutylenes, notably Indopol H-100 (molar mass or Mw = 965 g/mol), Indopol H-300 (Mw = 1340 g/mol) and Indopol H-1500 (Mw = 2160 g/mol) sold or manufactured by the company Amoco,
- polyisobutenes and hydrogenated polyisobutenes, notably Parleam^{®} sold by the company Nippon Oil Fats, Panalane H-300 E sold or manufactured by the company Amoco (Mw = 1340 g/mol), Viseal 20000 sold or manufactured by the company Synteal (Mw = 6000 g/mol) and Rewopal PIB 1000 sold or manufactured by the company Witco (Mw = 1000 g/mol),
- decene/butene copolymers and polybutene/polyisobutene copolymers, notably Indopol L-14,
- polydecenes and hydrogenated polydecenes, notably Puresyn 10 (Mw = 723 g/mol) and Puresyn 150 (Mw = 9200 g/mol) sold or manufactured by the company Mobil Chemicals,
- and mixtures thereof.

Said non-volatile oil may also be a diester or triester oil.

The ester oils may notably be hydroxylated.

The non-volatile ester oil may preferably be chosen from:
- diesters, notably comprising between 18 and 60 carbon atoms in total and in particular between 18 and 50 carbon atoms in total. It is notably possible to use diesters of dicarboxylic acids and of monoalcohols, preferably such as diisostearyl malate, or glycol diesters of monocarboxylic acids, such as neopentyl glycol diheptanoate or polyglyceryl-2 diisostearate (notably such as the compound sold under the trade reference Dermol DGDIS by the company Alzo);
- triesters, notably comprising between 35 and 70 carbon atoms in total, in particular such as triesters of a tricarboxylic acid, such as triisostearyl citrate, or tridecyl trimellitate, or glycol triesters of monocarboxylic acids such as polyglyceryl-2 triisostearate;
- tetraesters, notably with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or pentaerythrityl tetrakis(2-decyl)tetradecanoate;
- polyesters obtained by condensation of unsaturated fatty acid dimer and/or trimer and of diol, such as those described in patent application FR 0 853 634, in particular such as of dilinoleic acid and of 1,4-butanediol. Mention may notably be made in this respect of the polymer sold by Biosynthis under the name Viscoplast 14436H (INCI name: dilinoleic acid/butanediol copolymer), or else copolymers of polyols and of dimer diacids, and esters thereof, such as Hailuscent ISDA;
- esters and polyesters of diol dimer and of monocarboxylic or dicarboxylic acid, such as esters of diol dimer and of fatty acid and esters of diol dimer and of dicarboxylic acid dimer, in particular which may be obtained from a dicarboxylic acid dimer derived in particular from the dimerization of an unsaturated fatty acid notably of C₈ to C₃₄, notably of C₁₂ to C₂₂, in particular of C₁₆ to C₂₀ and more particularly of C₁₈, such as esters of dilinoleic diacids and of dilinoleic diol dimers, for instance those sold by the company Nippon Fine Chemical under the trade names Lusplan DD-DA5^{®} and DD-DA7^{®};
- vinylpyrrolidone/1-hexadecene copolymers, for instance the product sold under the name Antaron V-216 (also known as Ganex V216) by the company ISP (Mw = 7300 g/mol);
- hydrocarbon-based plant oils such as fatty acid triglycerides (which are liquid at room temperature), notably of fatty acids containing from 7 to 40 carbon atoms, such as heptanoic or octanoic acid triglycerides or jojoba oil; mention may be made in particular of saturated triglycerides such as caprylic/capric triglyceride, glyceryl triheptanoate, glyceryl trioctanoate, and C₁₈-₃₆ acid triglycerides such as those sold under the reference Dub TGI 24 by Stéarinerie Dubois, and unsaturated triglycerides such as castor oil, olive oil, ximenia oil and pracaxi oil;
- and mixtures thereof.

The other fatty substances that may be present in the oily phase are, for example, fatty acids including from 8 to 30 carbon atoms, for instance stearic acid, lauric acid, palmitic acid and oleic acid; waxes, for instance lanolin, beeswax, carnauba wax or candelilla wax, paraffin wax, lignite wax or microcrystalline waxes, ceresin or ozokerite, and synthetic waxes, for instance polyethylene waxes and Fischer-Tropsch waxes; silicone resins such as trifluoromethyl-C₁-C₄-alkyl dimethicone and trifluoropropyl dimethicone; and silicone elastomers, for instance the products sold under the name KSG by the company Shin-Etsu, under the name Trefil or BY29 by the company Dow Coming, or under the name Gransil by the company Grant Industries.

These fatty substances may be chosen in a varied manner by a person skilled in the art in order to prepare a composition having the desired properties, for example in terms of consistency or texture.

Preferably, a composition according to the invention comprises a fatty phase containing at least one fatty substance.

According to a preferred embodiment, a composition according to the invention comprises at least one non-volatile hydrocarbon-based oil, and preferably at least one apolar hydrocarbon-based oil.

Preferably, a composition according to the invention comprises at least two non-volatile hydrocarbon-based oils and one non-volatile ester oil.

According to a particularly preferred embodiment, a composition according to the invention comprises at least one non-volatile fatty acid triglyceride, one non-volatile carbonate oil and one non-volatile ester oil.

According to a particularly preferred embodiment, a composition according to the invention comprises at least caprylic/capric triglyceride, dicaprylyl carbonate and diisopropyl sebacate.

In a preferred manner, the oily phase or fatty phase may be present in a composition according to the invention in a content of between 2.0% and 20% by weight, preferably between 3.0% and 15% by weight, more preferentially between 4.0% and 12% by weight, or even between 5.0% and 10% by weight, and even more preferentially between 6.0% and 8.0% by weight, relative to the total weight of the composition.

### Adjuvants

### Surfactant

According to a preferred embodiment, a composition according to the invention may also comprise at least one surfactant.

The surfactants may be chosen from nonionic, anionic, cationic and amphoteric surfactants, and mixtures thereof. Reference may be made to Kirk-Othmer's Encyclopedia of Chemical Technology, Volume 22, pages 333-432, 3rd Edition, 1979, Wiley, for the definition of the emulsifying properties and functions of surfactants, in particular pages 347-377 of this reference, for the anionic, amphoteric and nonionic surfactants.

### Nonionic surfactant

Preferably, the composition according to the invention comprises at least one nonionic surfactant.

The nonionic surfactants may notably be chosen from alkyl and polyalkyl esters of poly(ethylene oxide) or of poly(propylene oxide), oxyalkylenated alcohols, alkyl and polyalkyl ethers of poly(ethylene oxide) or of poly(propylene oxide), optionally polyoxyethylenated alkyl and polyalkyl esters of sorbitan, optionally polyoxyethylenated alkyl and polyalkyl ethers of sorbitan, alkyl and polyalkyl glycosides or polyglycosides, in particular alkyl and polyalkyl glucosides or polyglucosides, alkyl and polyalkyl esters of sucrose, optionally polyoxyethylenated alkyl and polyalkyl esters of glycerol, and optionally polyoxyethylenated alkyl and polyalkyl ethers of glycerol, gemini surfactants, cetyl alcohol, stearyl alcohol, and mixtures thereof.

Oxyalkylenated, in particular oxyethylenated and/or oxypropylenated, alcohols that are preferably used are those that may include from 1 to 150 oxyethylene and/or oxypropylene units, in particular containing from 20 to 100 oxyethylene units, in particular ethoxylated fatty alcohols, notably of C₈-C₂₄ and preferably of C₁₂-C₁₈; those which may or may not be ethoxylated, for instance stearyl alcohol ethoxylated with 20 oxyethylene units (CTFA name Steareth-20), for instance Brij^{®} 78 sold by the company Uniqema, cetearyl alcohol ethoxylated with 30 oxyethylene units (CTFA name Ceteareth-30), and the mixture of C₁₂-C₁₅ fatty alcohols including 7 oxyethylene units (CTFA name C₁₂₋₁₅ Pareth-7), for instance the product sold under the name Neodol 25-7^{®} by Shell Chemicals; or in particular oxyalkylenated (oxyethylenated and/or oxypropylenated) alcohols containing from 1 to 15 oxyethylene and/or oxypropylene units, in particular ethoxylated C₈-C₂₄ and preferably C₁₂-C₁₈ fatty alcohols, such as stearyl alcohol ethoxylated with 2 oxyethylene units (CTFA name Steareth-2), for instance Brij^{®} 72 sold by the company Uniqema.

Optionally polyoxyethylenated alkyl and polyalkyl esters of sorbitan that are preferably used include those with a number of ethylene oxide (EO) units ranging from 0 to 100. Examples that may be mentioned include sorbitan laurate 4 or 20 EO, in particular polysorbate 20 (or polyoxyethylene (20) sorbitan monolaurate) such as the product Tween^{®} 20 sold by the company Uniqema, or polysorbate 60, sorbitan palmitate 20 EO, sorbitan isostearate, sorbitan stearate 20 EO, sorbitan oleate 20 EO, or else the Cremophor^{®} products (RH 40, RH 60, etc.) from BASF. The mixture of sorbitan stearate and of sucrose cocoate, sold under the name Arlacel^{®} 2121U-FL from Croda, may also be mentioned.

Alkyl and polyalkyl glucosides or polyglucosides that are preferably used include those containing an alkyl group including from 6 to 30 carbon atoms and preferably from 6 to 18 or even from 8 to 16 carbon atoms, and containing a glucoside group preferably comprising from 1 to 5 and notably 1, 2 or 3 glucoside units. The alkylpolyglucosides may be chosen, for example, from decylglucoside (alkyl-C₉/C₁₁-polyglucoside (1.4)), for instance the product sold under the name Mydol 10^{®} by the company Kao Chemicals or the product sold under the name Plantacare 2000 UP^{®} by the company Henkel and the product sold under the name Oramix NS 10^{®} by the company SEPPIC; caprylyl/capryl glucoside, for instance the product sold under the name Plantacare KE 3711^{®} by the company Cognis or Oramix CG 110^{®} by the company SEPPIC; laurylglucoside, for instance the product sold under the name Plantacare 1200 UP^{®} by the company Henkel or Plantaren 1200 N^{®} by the company Henkel; cocoyl glucoside, for instance the product sold under the name Plantacare 818 UP^{®} by the company Henkel; caprylyl glucoside, for instance the product sold under the name Plantacare 810 UP^{®} by the company Cognis; the mixture of arachidyl glucosyl and behenyl alcohol and arachidyl alcohol, the INCI name of which is Arachidyl alcohol (and) behenyl alcohol (and) arachidyl glucoside, sold under the name Montanov^{®} 202 by the company SEPPIC; and mixtures thereof.

As alkyl- and polyalkyl esters of glycerol, mention may be made of C₈-C₂₄, in particular C₁₂-C₂₂ fatty acid esters of glycerol, such as the glycerol ester of isooctadecanoic acid (CTFA name: Glyceryl isostearate).

As alkyl- and polyalkyl ethers of poly(ethylene oxide) or of poly(propylene oxide), mention may be made of the compounds PPG-6-Decyltetradeceth-30, PPG-6-Decyltetradeceth-12, PPG-13-Decyltetradeceth-24 , PPG-6-Decyltetradeceth-20, PPG-4 Ceteth-1, PPG-8 Ceteth-1, PPG-4 Ceteth- 10, PPG-4 Ceteth-20, PPG-5 Ceteth-20, PPG-8 Ceteth-20, and PPG-23 Steareth-34.

### Anionic surfactant

The anionic surfactants may be chosen from alkyl ether sulfates, carboxylates, amino acid derivatives, sulfonates, isethionates, taurates, sulfosuccinates, alkylsulfoacetates, phosphates and alkyl phosphates, polypeptides, metal salts of C₁₀-C₃₀ and notably C₁₆-C₂₅ fatty acids, in particular metal stearates and behenates, and mixtures thereof.

### Cationic surfactant

The cationic surfactants may be chosen from alkylimidazolidiniums, such as isostearyl ethylimidonium ethosulfate, ammonium salts such as (C₁₂-₃₀-alkyl)-tri(C₁₋₄₋alkyl)ammonium halide such as N,N,N-trimethyl-1-docosanaminium chloride (or behentrimonium chloride).

### Amphoteric surfactant

The compositions according to the invention may also contain one or more amphoteric surfactants, for instance N-acylamino acids such as N-alkyl aminoacetates and disodium cocoamphodiacetate, and amine oxides such as stearamine oxide, or alternatively silicone surfactants, for instance dimethicone copolyol phosphates such as the product sold under the name Pecosil PS 100^{®} by the company Phoenix Chemical.

### Silicone surfactant

The composition may also comprise at least one silicone surfactant. By way of example, as nonionic surfactants with an HLB of greater than or equal to 8 at 25°C, used alone or as a mixture, mention may be made of dimethicone copolyol or dimethicone copolyol benzoate, and as nonionic surfactants with an HLB of less than 8 at 25°C, used alone or as a mixture, mention may be made of a cyclomethicone/dimethicone copolyol mixture.

The surfactant(s) may be present in a composition according to the invention in a proportion ranging from 0.01% to 5% by weight and preferably from 0.5% to 2.5% by weight relative to the total weight of the composition.

### Fillers

A composition according to the invention may also comprise at least one filler.

The filler may be chosen from pigments, titanium oxide, red iron oxide, yellow iron oxide, black iron oxide, boron nitride, nacres, synthetic or natural mica, nacres comprising mica and titanium oxide, silica powder, talc, polyamide particles and notably those sold under the name Orgasol^{®} by the company Atochem, polyethylene powders, microspheres based on acrylic copolymers, such as those made of ethylene glycol dimethacrylate/lauryl methacrylate copolymer sold by the company Dow Coming under the name Polytrap^{®}, expanded powders such as hollow microspheres and notably the microspheres sold under the name Expancel^{®} by the company Kemanord Plast or under the name Micropearl^{®} F 80 ED by the company Matsumoto, silicone resin microbeads such as those sold under the name Tospearl^{®} by the company Toshiba Silicone, and mixtures thereof.

Preferably, the composition according to the invention comprises boron nitride.

These fillers may be present in a composition according to the invention in a content ranging from 0.1% to 5.0% by weight and preferably from 1.0% to 3.0% by weight relative to the total weight of the composition.

### Active agents

A composition according to the invention may comprise additional active agents, in particular antiaging active agents other than the retinol used according to the invention. By way of example of antiaging active agents, mention may be made of n-octanoyl-5-salicylic acid, adenosine, c-beta-d-xylopyranoside-2-hydroxypropane and the sodium salt of 3-hydroxy-2-pentylcyclopentyl)acetic acid.

Such active agents may be present in a composition according to the invention in a content ranging from 0.05% to 5.0% by weight and preferably from 0.05% to 1.5% by weight relative to the total weight of the composition.

According to a preferred embodiment, a composition according to the invention is preferably free of compounds which may be harmful to human beings and/or the environment, i.e. it comprises less than 0.2% by weight, in particular less than 0.1% by weight, preferably less than 0.05% by weight, and more preferentially less than 0.01% by weight, or even is totally free of compounds which may be harmful to human beings and/or the environment, in particular free of butyl hydroxytoluene (BHT) and/or ethylenediaminetetraacetic acid (EDTA) or a salt thereof.

Thus, a composition according to the invention is in particular free of butyl hydroxytoluene (BHT) and/or ethylenediaminetetraacetic acid (EDTA) or a salt thereof, and preferably is totally free of ethylenediaminetetraacetic acid or a salt thereof.

A composition according to the invention may also include at least one additive chosen from the adjuvants conventionally used in the cosmetic field, such as preserving agents, fragrances, dyestuffs, polar additives, film-forming polymers, pH modifiers (acids or bases), cosmetic active agents, for instance moisturizers, cicatrizing agents, agents for combating greasy skin, and/or anti-pollution agents, and mixtures thereof.

Needless to say, a person skilled in the art will take care to select this or these optional additional compound(s), and/or the amount thereof, such that the advantageous properties of a composition according to the invention are not, or are not substantially, adversely affected by the envisioned addition.

### Composition

As stated previously, a composition according to the invention may be cosmetic and/or dermatological, and is preferably cosmetic.

A composition according to the invention is generally suitable for topical application to the skin and thus generally comprises a physiologically acceptable medium, i.e. a medium that is compatible with the skin.

It is preferably a cosmetically acceptable medium, i.e. a medium which has a pleasant color, odor and feel and which does not cause any unacceptable discomfort, i.e. stinging, tautness or redness, liable to discourage the consumer from applying this composition.

A composition according to the invention is in the form of an oil-in-water emulsified gel.

In particular, a composition according to the invention has a pH ranging from 3 to 8. Preferably, the pH of the composition ranges from 4 to 7 and more preferentially from 5 to 7.

A composition according to the invention may be prepared according to the techniques that are well known to those skilled in the art.

### Intended use of the composition

A composition according to the invention may be in the form of a cosmetic composition for caring for and/or making up keratin materials, in particular of the body or of the face, preferably of the face.

These compositions may constitute cleansing, protective, treating or care creams for the face, the hands or the body, for example day creams, night creams, makeup creams, foundation creams, antisun creams, fluid foundations, protective or care body milks, antisun milks, skincare gels or foams, for instance bath compositions, deodorant compositions comprising a bactericidal agent, aftershave gels, or hair-removing creams or blister treatment products.

Thus, a cosmetic composition according to the invention may be used as a care product and/or antisun product for the face and/or body, with a liquid to semiliquid consistency, such as milks, more or less smooth creams, gel-creams or pastes.

Preferably, a composition according to the invention is in the form of a cosmetic composition for caring for keratin materials, in particular the skin of the body or the face, preferably of the face.

In particular, a composition of the invention may be in the form of an antiaging care composition for the skin of the body or the face, in particular the face.

According to another embodiment, a composition of the invention may be in the form of a composition for making up keratin materials, in particular of the body or of the face, preferably of the face.

Thus, according to a submode of this embodiment, a composition of the invention may be in the form of a makeup base composition for making up. A composition of the invention may in particular be in the form of a foundation.

According to yet another submode of this embodiment, a composition of the invention may be in the form of a lip product, notably a lipstick.

According to yet another submode of this embodiment, a composition of the invention may be in the form of a product for the eyebrows, in particular an eyebrow pencil.

Such compositions are notably prepared according to the general knowledge of a person skilled in the art.

Thus, the invention also relates to the use of a composition according to the invention for caring for and/or making up keratin materials, preferably for caring for keratin materials, in particular the skin of the body and/or of the face.

The invention also relates to a cosmetic process for making up and/or caring for keratin materials, in particular the skin and/or the lips, comprising at least one step of applying a composition as defined previously to said keratin materials.

Preferably, the invention also relates to a cosmetic process for caring for keratin materials, in particular the skin and/or the lips, comprising at least one step of applying a composition as defined above to said keratin materials.

Said cosmetic processes for making up and/or caring for keratin materials, in particular the skin and/or the lips, are non-therapeutic.

In particular, a composition according to the invention may be used for combating the signs of skin aging.

Thus, the present application also relates to the use of a composition according to the invention for combating the signs of skin aging.

The composition may be applied to the skin by hand or using an applicator.

Throughout the description, including the claims, the expression *"including a"* should be understood as being synonymous with *"including at least one",* unless otherwise specified. The terms *"between... and...", "comprises from* ... *to...",* "*formed from* ... *to..."* and *"ranging from... to..."* should be understood as being inclusive of the limits, unless otherwise specified.

The invention is illustrated in greater detail by the examples presented below. Unless otherwise indicated, the amounts shown are expressed as mass percentages.

### Example

### Example 1

The antiaging care compositions 1 outside the invention, and 2 according to the invention are prepared using the weight proportions as detailed in the table 1 below. The values are expressed as weight percentages relative to the total weight of the composition.

### [Table 1]

**Table 1**

| **Phase** | **Compounds (INCI name) (Trade name)** | **Formula 1 outside the invention** | **Formula 2 according to the invention** |
|---|---|---|---|
| Phase A | Water | qs 100 | qs 100 |
| | Glycerol (*BP glycerol from Organic Chemical Corporation)* | 11 | 11 |
| | Propylene glycol (*monopropylene glycol from Repsol)* | 2 | 2 |
| | Pentylene glycol (*Pentiol from Minasolve*) | 3 | 3 |
| | Butylene glycol (*1,3-butylene glycol*) | 0.5 | 0.5 |
| | Caprylyl glycol (*Hydrolite^{®} CG from Symrise)* | 0.2 | 0.2 |
| | Citric acid (*fine citric acid monohydrate F6000)* | / | 0.01 |
| | Sodium hyaluronate | 0.2 | 0.2 |
| | Trisodium ethylenediamine disuccinate (containing 37% active material) (*Natrlquest^{®} E30 from Innospec Active Chemicals)* | / | 0.2 |
| | Disodium EDTA (*Edeta^{®} BD from BASF)* | 0.15 | / |
| | Phenoxyethanol (*Phenoxetol*^{™} *from Clariant)* | 0.4 | 0.4 |
| Phase B | Ammonium polyacryloyldimethyltaurate (*Hostacerin*^{®} *AMPS from Clariant*) | 0.2 | 0.2 |
| | Xanthan gum (*Keltrol*^{®} *CG-T from CP Kelco)* | 0.2 | 0.2 |
| Phase C | PPG-6-decyltetradeceth-30 (*Nikkol*^{®} *PEN-4630 from Nikkol*) | 0.8 | 0.8 |
| Phase D | Pentaerythrityl tetra-di-t-butyl hydroxyhydrocinnamate (*Tinoguard*^{®} *TT from BASF)* | 0.1 | 0.1 |
| | Dicaprylyl carbonate (*Cetiol*^{®} *CC from BASF)* | 1 | 1 |
| Phase E | Isononyl isononanoate (*isononyl isononanoate from DUB Inin from Stéarinerie Dubois)* | 3 | 3 |
| | PEG-6 caprylic/capric glycerides *(Softigen^{®} 767 from IOI Oleo GmbH*) | 1 | 1 |
| | Dicaprylyl ether (*Cetiol*^{®} *OE from BASF*) | 3 | 3 |
| | Glyceryl isostearate (*Peceol*^{®} *Isostearique from Gattefossé*) | 0.3 | 0.3 |
| | Retinol (10% active material) (*Retinol 10SU from BASF*) | 3.5 | 3.5 |
| Phase F | Sodium hydroxide | 0.011 | / |
| Phase G | Ethanol (*absolute alcohol*) | 8 | 8 |
| **pH** | | **6.5** | **6.9** |
| **Rheomat viscosity at 25°C** | | **45 UD** | **54 UD** |

### Protocol for preparing the compositions

The compositions are prepared using a Rotor/Stator, according to the following process:
The aqueous phase A is introduced into a beaker until the ingredients are fully dissolved. The gelling agents are added, starting with ammonium polyacryloyldimethyl taurate, until the gel is totally hydrated, then the other gelling agents, namely xanthan gum and sodium hyaluronate, hydrated in part of the glycerol, are added until fully dissolved and hydrated.
In parallel, PPG-6-decyltetradeceth-30 is dissolved hot (65-70°C) in the remaining glycerol and is then introduced into the beaker. Similarly, pentaerythrityl tetra-di-t-butyl hydroxyhydrocinnamate is dissolved hot in dicaprylyl carbonate. The whole mixture is then emulsified with the oily phase E. The pH is adjusted by addition of sodium hydroxide, and ethanol is then added.

In contrast with composition 1 outside the invention, composition 2 according to the invention has a pleasant odor. Composition 1 outside the invention has a fatty and rancid odor. The same phenomenon is observed after one month of daily use of the two formulations and also after 2 months of storage at room temperature and at 45°C.

### Example 2

The antiaging care compositions 3 to 5 according to the invention are prepared, using the weight proportions as detailed in the table 2 below, as indicated in example 1. The values are expressed as weight percentages relative to the total weight of the composition.

### [Table 2]

**Table 2**

| **Phase** | **INCI Compounds** | **Formula 3 according to the invention** | **Formula 4 according to the invention** | **Formula 5 according to the invention** |
|---|---|---|---|---|
| Phase A | Water | qs 100 | qs 100 | qs 100 |
| | Glycerol (*BP glycerol from Organic Chemical Corporation*) | 11 | 11 | 11 |
| | Propylene glycol *(monopropylene glycol from Repsol*) | 2 | 2 | 2 |
| | Pentylene glycol (*Pentiol from Minasolve)* | 3 | 3 | 3 |
| | Butylene glycol (*1,3-butylene glycol*) | 0.5 | 0.5 | 0.5 |
| | Caprylyl glycol (*Hydrolite*^{®} *CG from Symrise*) | 0.2 | 0.2 | 0.2 |
| | PEG-8 (*Pluracare*^{®} *E 400 from BASF*) | / | / | 4 |
| | Citric acid (*fine citric acid monohydrate F6000*) | 0.015 | 0.015 | 0.015 |
| | Sodium hyaluronate | 0.2 | 0.2 | 0.2 |
| | Trisodium ethylenediamine disuccinate (containing 37% active material) (*Natrlquest*^{®} *E30 from Innospec Active Chemicals*) | 0.2 | 0.2 | 0.2 |
| | Phenoxyethanol (*Phenoxetol*^{™} *from Clariant*) | 0.4 | 0.4 | 0.4 |
| Phase B | Ammonium polyacryloyldimethyltaurate (*Hostacerin*^{®} *AMPS from Clariant*) | 0.2 | 0.2 | 0.2 |
| | Xanthan gum (Keltrol^{®} CG-T from CP Kelco) | 0.2 | 0.2 | / |
| | Xanthan gum (and) sclerotium gum (and) lecithin (and) pullulan (*Siligel*^{®} *from Lucas Meyer Cosmetics*) | / | / | 0.2 |
| Phase C | PPG-6-decyltetradeceth-30 (*Nikkol*^{®} *PEN-4630 from Nikkol*) | 0.8 | 0.8 | 0.8 |
| Phase D | Pentaerythrityl tetra-di-t-butyl hydroxyhydrocinnamate (*Tinoguard*^{®} *TT from BASF*) | 0.1 | 0.1 | 0.1 |
| | Dicaprylyl carbonate (*Cetiol*^{®} *CC from BASF*) | 1 | 1 | 1 |
| Phase E | Isononyl isononanoate (*isononyl isononanoate from DUB Inin from Stéarinerie Dubois*) | 3 | 3 | 3 |
| | PEG-6 caprylic/capric glycerides (*Softigen*^{®} *767 from IOI Oleo GmbH)* | 1 | 1 | 1 |
| | Dicaprylyl ether (*Cetiol*^{®} *OE from BASF*) | 3 | 3 | 3 |
| | Dimethicone (and) polysilicone-11 (*Gransil*^{®} *RPS-D6 from Grant Industries*) | / | / | 2 |
| | Glyceryl isostearate (*Peceol*^{®} *Isostearique from Gattefossé*) | 0.3 | 0.3 | 0.3 |
| | Fragrance | / | 0.2 | 0.3 |
| | Retinol (10% active material) (*Retinol 10SU from BASF*) | 3.5 | 2.5 | 2.5 |
| Phase F | Ethanol (*absolute alcohol*) | 8 | 8 | 8 |

Compositions 3 to 5 according to the invention have a pleasant odor. In particular, the compositions according to the invention have a pleasant odor. The same phenomenon is observed after one month of daily use of the two formulations and also after 2 months of storage at room temperature and at 45°C.

## Claims

1. A composition, notably a cosmetic composition, in particular for making up and/or caring for keratin materials, in the form of an oil-in-water emulsified gel, comprising:
- retinol;
- di-t-butyl pentaerythrityl tetrahydroxycinnamate;
- at least one ethylenediaminedisuccinic acid salt;
- at least 11% by weight of at least one polyol relative to the total weight of the composition;
- at least one hydrophilic gelling agent;
- at least one derivative of polyethylene glycol and of mono-, di- and triglycerides of an acid including at least one C₆ to C₁₆ alkyl chain, and containing at least two ethylene oxide groups;
- at least one non-volatile ether oil containing from 10 to 40 carbon atoms; and
- at least one non-volatile monoester oil.

2. The composition as claimed in the preceding claim, comprising from 0.02% to 5.0% by weight, preferably from 0.05% to 3.0% by weight, notably from 0.08% to 1.0%, and more preferentially from 0.1% to 0.5% by weight of retinol, relative to the total weight of the composition.

3. The composition as claimed in any of the preceding claims, comprising from 0.01% to 2.5% by weight, preferably from 0.05% to 1.5% by weight, more preferentially from 0.1% to 0.3% by weight of di-t-butyl pentaerythrityl tetrahydroxycinnamate, relative to the total weight of the composition.

4. The composition as claimed in any of the preceding claims, comprising from 0.01% to 2.5% by weight, preferably from 0.05% to 1.5% by weight, more preferentially from 0.07% to 0.3% by weight of ethylenediaminedisuccinic acid salt, relative to the total weight of the composition.

5. The composition as claimed in any of the preceding claims, in which the ethylenediaminedisuccinic acid salt is chosen from the potassium, sodium and ammonium salts and the amine salts of ethylenediaminedisuccinic acid, and preferably is trisodium ethylenediaminedisuccinate.

6. The composition as claimed in any of the preceding claims, in which the mass ratio of di-t-butyl pentaerythrityl tetrahydroxycinnamate / ethylenediaminedisuccinic acid salt is between 1.5 and 5, and preferably between 2.5 and 4.5.

7. The composition as claimed in any of the preceding claims, comprising from 11% to 25% by weight, preferably from 13% to 20% by weight, more preferentially from 14% to 18% by weight of polyol(s), relative to the total weight of the composition.

8. The composition as claimed in any of the preceding claims, **characterized in that** the polyol(s) are chosen from ethylene glycol, propylene glycol, 1,3-propanediol, 1,3-butylene glycol, isoprene glycol, 1,2-pentylene glycol, hexylene glycol, caprylyl glycol, dipropylene glycol, glycerol, pentaerythritol, trimethylolpropane, polyglycerols, polyethylene glycols, and mixtures thereof.

9. The composition as claimed in any of the preceding claims, **characterized in that** it comprises at least two different polyols, and preferably at least three different polyols.

10. The composition as claimed in any of the preceding claims, **characterized in that** it comprises at least glycerol and at least one different polyol chosen from ethylene glycol, propylene glycol, 1,3-propanediol, 1,3-butylene glycol, isoprene glycol, 1,2-pentylene glycol, hexylene glycol, caprylyl glycol, dipropylene glycol, and mixtures thereof.

11. The composition as claimed in any of the preceding claims, **characterized in that** the hydrophilic gelling agent(s) are chosen from crosslinked and/or neutralized 2-acrylamido-2-methylpropanesulfonic acid polymers and copolymers, polysaccharides, and mixtures thereof.

12. The composition as claimed in any of the preceding claims, **characterized in that** it comprises at least two different hydrophilic gelling agents.

13. The composition as claimed in any of the preceding claims, in which the derivative of polyethylene glycol and of mono-, di- and tri-glycerides of an acid including at least one C₆ to C₁₆ alkyl chain, and containing at least two ethylene oxide groups is chosen from the derivatives of polyethylene glycol and of mono-, di- and tri-glycerides of an acid including at least one C₆ to C₁₄ alkyl chain, and containing from 6 to 10 ethylene oxide groups, and preferably from derivatives of polyethylene glycol and of mono-, di- and triglycerides of caprylic acid and of capric acid containing 6 ethylene oxide groups.

14. The composition as claimed in any of the preceding claims, in which the non-volatile ether oil containing from 10 to 40 carbon atoms is dicapryl ether.

15. The composition as claimed in any of the preceding claims, in which the non-volatile monoester oil is chosen from isononyl isononanoate, oleyl erucate and/or octyl-2-dodecyl neopentanoate, and preferably is isononyl isononanoate.

16. The composition as claimed in any of the preceding claims, **characterized in that** it also comprises a non-volatile oil other than the non-volatile ether oil containing from 10 to 40 carbon atoms and the non-volatile monoester oil, and in particular chosen from nonvolatile oils of carbonate type, and preferably is dicaprylyl carbonate.

17. The composition as claimed in any of the preceding claims, **characterized in that** the fatty phase is present in a content of between 2.0% and 20% by weight, preferably between 3.0% and 15% by weight, more preferentially between 4.0% and 12% by weight, or even between 5.0% and 10% by weight, and even more preferentially between 6.0% and 8% by weight, relative to the total weight of the composition.

18. The composition as claimed in any of the preceding claims, **characterized in that** it also comprises at least one surfactant, preferably chosen from nonionic surfactants.

19. A non-therapeutic cosmetic process for making up and/or caring for, preferably for caring for, keratin materials, in particular the skin and/or the lips, comprising at least one step of applying a composition as defined in any of the preceding claims to said keratin materials.

## Patentansprüche

1. Zusammensetzung, insbesondere eine Kosmetikzusammensetzung, speziell zum Zurechtmachen und/oder Pflegen von Keratinmaterialien, in Form eines emulgierten Ölin-Wasser-Gels, umfassend:
- Retinol;
- Di-t-butylpentaerythrityltetrahydroxycinnamat;
- mindestens ein Ethylendiamindibernsteinsäuresalz;
- mindestens 11 Gew.% von mindestens einem Polyol, bezogen auf das Gesamtgewicht der Zusammensetzung;
- mindestens ein hydrophiles Geliermittel;
- mindestens ein Derivat von Polyethylenglykol und Mono-, Di- und Triglyceriden einer Säure, die mindestens eine C₆- bis C₁₆-Alkylkette einschließt, und das mindestens zwei Ethylenoxidgruppen enthält;
- mindestens ein nicht-flüchtiges Etheröl, das 10 bis 40 Kohlenstoffatome enthält; und
- mindestens ein nicht-flüchtiges Monoesteröl.

2. Zusammensetzung nach dem vorhergehenden Anspruch, umfassend 0,02 Gew.% bis 5,0 Gew.%, vorzugsweise 0,05 Gew.% bis 3,0 Gew.%, insbesondere 0,08 Gew.% bis 1,0 Gew.% und bevorzugter 0,1 Gew.% bis 0,5 Gew.% Retinol, bezogen auf das Gesamtgewicht der Zusammensetzung.

3. Zusammensetzung nach einem der vorhergehenden Ansprüche, umfassend 0,01 Gew.% bis 2,5 Gew.%, vorzugsweise 0,05 Gew.% bis 1,5 Gew.%, bevorzugter 0,1 Gew.% bis 0,3 Gew.% Di-t-butylpentaerythrityltetrahydroxycinnamat, bezogen auf das Gesamtgewicht der Zusammensetzung.

4. Zusammensetzung nach einem der vorhergehenden Ansprüche, umfassend 0,01 Gew.% bis 2,5 Gew.%, vorzugsweise 0,05 Gew.% bis 1,5 Gew.%, bevorzugter 0,07 Gew.% bis 0,3 Gew.% Ethylendiamindibernsteinsäuresalz, bezogen auf das Gesamtgewicht der Zusammensetzung.

5. Zusammensetzung nach einem der vorhergehenden Ansprüche, wobei das Ethylendiamindibernsteinsäuresalz ausgewählt ist aus Kalium-, Natrium- und Ammoniumsalzen und den Aminsalzen von Ethylendiamindibernsteinsäure, und vorzugsweise Trinatriumethylendiamindisuccinat ist.

6. Zusammensetzung nach einem der vorhergehenden Ansprüche, wobei das Massenverhältnis von Di-t-butylpentaerythrityltetrahydroxycinnamat / Ethylendiamindibernsteinsäuresalz zwischen 1,5 und 5 und vorzugsweise zwischen 2,5 und 4,5 liegt.

7. Zusammensetzung nach einem der vorhergehenden Ansprüche, umfassend 11 Gew.% bis 25 Gew.%, vorzugsweise 13 Gew.% bis 20 Gew.%, bevorzugter 14 Gew.% bis 18 Gew.% Polyol(e), bezogen auf das Gesamtgewicht der Zusammensetzung.

8. Zusammensetzung nach einem der vorhergehenden Ansprüche, **dadurch gekennzeichnet, dass** das Polyol/die Polyole ausgewählt ist/sind aus Ethylenglykol, Propylenglykol, 1,3-Propandiol, 1,3-Butylenglykol, Isoprenglykol, 1,2-Pentylenglykol, Hexylenglykol, Caprylylglykol, Dipropylenglykol, Glycerin, Pentaerythritol, Trimethylolpropan, Polyglycerinen, Polyethylenglykolen und Mischungen davon.

9. Zusammensetzung nach einem der vorhergehenden Ansprüche, **dadurch gekennzeichnet, dass** sie mindestens zwei unterschiedliche Polyole und vorzugsweise mindestens drei unterschiedliche Polyole umfasst.

10. Zusammensetzung nach einem der vorhergehenden Ansprüche, **dadurch gekennzeichnet, dass** sie mindestens Glycerin und mindestens ein anderes Polyol ausgewählt aus Ethylenglykol, Propylenglykol, 1,3-Propandiol, 1,3-Butylenglykol, Isoprenglykol, 1,2-Pentylenglykol, Hexylenglykol, Caprylylglykol, Dipropylenglykol und Mischungen davon umfasst.

11. Zusammensetzung nach einem der vorhergehenden Ansprüche, **dadurch gekennzeichnet, dass** das/die hydrophile Geliermittel ausgewählt ist/sind aus vernetzten und/oder neutralisierten 2-Acrylamido-2-me-thylpropansulfonsäurepolymeren und -copolymeren, Polysacchariden und Mischungen davon.

12. Zusammensetzung nach einem der vorhergehenden Ansprüche, **dadurch gekennzeichnet, dass** sie mindestens zwei unterschiedliche hydrophile Geliermittel umfasst.

13. Zusammensetzung nach einem der vorhergehenden Ansprüche, wobei das Derivat von Polyethylenglykol und Mono-, Di- und Triglyceriden einer Säure, die mindestens eine C6- bis C16-Alkylkette einschließt, und das mindestens zwei Ethylenoxidgruppen enthält, ausgewählt ist aus den Derivaten von Polyethylenglykol und Mono-, Di- und Triglyceriden von einer Säure, die mindestens eine C6- bis C14-Alkylkette einschließt, und die 6 bis 10 Ethylenoxidgruppen enthalten, und vorzugsweise aus Derivaten von Polyethylenglykol und von Mono-, Di- und Triglyceriden von Caprylsäure und von Caprinsäure, die 6 Ethylenoxidgruppen enthalten.

14. Zusammensetzung nach einem der vorhergehenden Ansprüche, wobei das nicht-flüchtige Etheröl, das 10 bis 40 Kohlenstoffatome enthält, Dicaprylether ist.

15. Zusammensetzung nach einem der vorhergehenden Ansprüche, wobei das nicht-flüchtige Monoesteröl ausgewählt ist aus Isononylisononanoat, Oleylerucat und/oder Octyl-2-dodecylneopentanoat und vorzugsweise Isononylisononanoat ist.

16. Zusammensetzung nach einem der vorhergehenden Ansprüche, **dadurch gekennzeichnet, dass** sie auch ein nicht-flüchtiges Öl umfasst, das von dem nicht-flüchtigen Etheröl, das 10 bis 40 Kohlenstoffatome enthält, und dem nicht-flüchtigen Monoesteröl verschieden ist, und insbesondere ausgewählt ist aus nicht-flüchtigen Ölen vom Carbonattyp, und das vorzugsweise Dicaprylylcarbonat ist.

17. Zusammensetzung nach einem der vorhergehenden Ansprüche, **dadurch gekennzeichnet, dass** die Fettphase in einem Gehalt zwischen 2,0 Gew.% und 20 Gew.% vorhanden ist, vorzugsweise zwischen 3,0 Gew.% und 15 Gew.%, bevorzugter zwischen 4,0 Gew.% und 12 Gew.%, oder sogar zwischen 5,0 Gew.% und 10 Gew.% und noch bevorzugter zwischen 6,0 Gew.% und 8 Gew.%, bezogen auf das Gesamtgewicht der Zusammensetzung.

18. Zusammensetzung nach einem der vorhergehenden Ansprüche, **dadurch gekennzeichnet, dass** sie auch mindestens ein Tensid umfasst, das vorzugsweise ausgewählt ist aus nicht-ionischen Tensiden.

19. Nicht-therapeutisches kosmetisches Verfahren zum Zurechtmachen und/oder Pflegen von Keratinmaterialien, vorzugsweise zum Pflegen von Keratinmaterialien, insbesondere von der Haut und/oder den Lippen, umfassend mindestens einen Schritt des Auftragens einer Zusammensetzung wie in einem der vorhergehenden Ansprüche definiert auf die Keratinmaterialien.

## Revendications

1. Composition, notamment composition cosmétique, en particulier pour le maquillage et/ou le soin des matières kératiniques, sous la forme d'un gel émulsifié de type huile-dans-eau, comprenant :
- du rétinol ;
- du tétrahydroxycinnamate de di-t-butyle pentaérythrityle ;
- au moins un sel d'acide éthylènediaminedisuccinique ;
- au moins 11 % en poids d'au moins un polyol par rapport au poids total de la composition ;
- au moins un agent gélifiant hydrophile ;
- au moins un dérivé de polyéthylène glycol et de monoglycérides, diglycérides et triglycérides d'un acide comprenant au moins une chaîne alkyle en C₆ à C₁₆, et contenant au moins deux groupes oxyde d'éthylène ;
- au moins une huile d'éther non volatile contenant de 10 à 40 atomes de carbone ; et
- au moins une huile de monoester non volatile.

2. Composition selon la revendication précédente, comprenant de 0,02 % à 5,0 % en poids, préférablement de 0,05 % à 3,0 % en poids, notamment de 0,08 % à 1,0 %, et plus préférentiellement de 0,1 % à 0,5 % en poids de rétinol, par rapport au poids total de la composition.

3. Composition selon l'une quelconque des revendications précédentes, comprenant de 0,01 % à 2,5 % en poids, préférablement de 0,05 % à 1,5 % en poids, plus préférentiellement de 0,1 % à 0,3 % en poids de tétrahydroxycinnamate de di-t-butyle pentaérythrityle, par rapport au poids total de la composition.

4. Composition selon l'une quelconque des revendications précédentes, comprenant de 0,01 % à 2,5 % en poids, préférablement de 0,05 % à 1,5 % en poids, plus préférentiellement de 0,07 % à 0,3 % en poids de sel d'acide éthylènediaminedisuccinique, par rapport au poids total de la composition.

5. Composition selon l'une quelconque des revendications précédentes, dans laquelle le sel d'acide éthylènediaminedisuccinique est choisi parmi les sels de potassium, de sodium et d'ammonium et les sels d'amine d'acide éthylènediaminedisuccinique, et préférablement est l'éthylènediaminedisuccinate trisodique.

6. Composition selon l'une quelconque des revendications précédentes, dans laquelle le rapport en masse de tétrahydroxycinnamate de di-t-butyle pentaérythrityle/sel d'acide éthylènediaminedisuccinique est compris entre 1,5 et 5, et préférablement entre 2,5 et 4,5.

7. Composition selon l'une quelconque des revendications précédentes, comprenant de 11 % à 25 % en poids, préférablement de 13 % à 20 % en poids, plus préférentiellement de 14 % à 18 % en poids de polyol(s), par rapport au poids total de la composition.

8. Composition selon l'une quelconque des revendications précédentes, **caractérisée en ce que** le ou les polyols sont choisis parmi l'éthylèneglycol, le propylèneglycol, le 1,3-propanediol, le 1,3-butylèneglycol, l'isoprène glycol, le 1,2-pentylène glycol, l'hexylèneglycol, le caprylyl-glycol, le dipropylèneglycol, le glycérol, le pentaérythritol, le triméthylolpropane, des polyglycérols, des polyéthylène glycols et des mélanges correspondants.

9. Composition selon l'une quelconque des revendications précédentes, **caractérisée en ce qu'**elle comprend au moins deux polyols différents, et préférablement au moins trois polyols différents.

10. Composition selon l'une quelconque des revendications précédentes, **caractérisée en ce qu'**elle comprend au moins du glycérol et au moins un polyol différent choisi parmi l'éthylèneglycol, le propylèneglycol, le 1,3-propanediol, le 1,3-butylèneglycol, l'isoprène glycol, le 1,2-pentylène glycol, l'hexylèneglycol, le caprylyl-glycol, le dipropylèneglycol et des mélanges correspondants.

11. Composition selon l'une quelconque des revendications précédentes, **caractérisée en ce que** l'agent ou les agents gélifiants hydrophiles sont choisis parmi des polymères et copolymères d'acide 2-acrylamido-2-méthylpropanesulfonique réticulés et/ou neutralisés, des polysaccharides et des mélanges correspondants.

12. Composition selon l'une quelconque des revendications précédentes, **caractérisée en ce qu'**elle comprend au moins deux agents gélifiants hydrophiles différents.

13. Composition selon l'une quelconque des revendications précédentes, dans laquelle le dérivé de polyéthylène glycol et de monoglycérides, diglycérides et triglycérides d'un acide comprenant au moins une chaîne alkyle en C₆ à C₁₆, et contenant au moins deux groupes oxyde d'éthylène est choisi parmi les dérivés de polyéthylène glycol et de monoglycérides, diglycérides et triglycérides d'un acide comprenant au moins une chaîne alkyle en C₆ à C₁₄, et contenant de 6 à 10 groupes oxyde d'éthylène, et préférablement parmi des dérivés de polyéthylène glycol et de monoglycérides, diglycérides et triglycérides d'acide caprylique et d'acide caprique contenant 6 groupes oxyde d'éthylène.

14. Composition selon l'une quelconque des revendications précédentes, dans laquelle l'huile d'éther non volatile contenant de 10 à 40 atomes de carbone est l'éther de dicapryle.

15. Composition selon l'une quelconque des revendications précédentes, dans laquelle l'huile de monoester non volatile est choisie parmi l'isononanoate d'isononyle, l'érucate d'oléyle et/ou le néopentanoate d'octyl-2-dodécyle, et préférablement est l'isononanoate d'isononyle.

16. Composition selon l'une quelconque des revendications précédentes, **caractérisée en ce qu'**elle comprend également une huile non volatile autre que l'huile d'éther non volatile contenant de 10 à 40 atomes de carbone et l'huile de monoester non volatile, et en particulier choisie parmi des huiles non volatiles de type carbonate, et préférablement est le carbonate de dicaprylyle.

17. Composition selon l'une quelconque des revendications précédentes, **caractérisée en ce que** la phase grasse est présente en une teneur comprise entre 2,0 % et 20 % en poids, préférablement entre 3,0 % et 15 % en poids, plus préférentiellement entre 4,0 % et 12 % en poids, ou même entre 5,0 % et 10 % en poids, et encore plus préférentiellement entre 6,0 % et 8 % en poids, par rapport au poids total de la composition.

18. Composition selon l'une quelconque des revendications précédentes, **caractérisée en ce qu'**elle comprend également au moins un tensioactif, préférablement choisi parmi des tensioactifs non ioniques.

19. Procédé cosmétique non thérapeutique pour le maquillage et/ou le soin, préférablement pour le soin, des matières kératiniques, en particulier la peau et/ou les lèvres, comprenant au moins une étape d'application d'une composition telle que définie dans l'une quelconque des revendications précédentes sur lesdites matières kératiniques.
